# EUROPEAN PATENT APPLICATION

(11) **EP 4 198 142 A1**
(43) Date of publication of application: **21.06.2023**
(21) Application number: 21855384.0
(22) Date of filing: 30.07.2021
(51) Int. Cl.: C12Q 1/68

(54) **KIT AND METHOD FOR DETECTING NUCLEIC ACID AND USES THEREOF**

(30) Priority: 11.08.2020 CN 202010803405
(71) Applicant: Shanghai Epiprobe Biotechnology Co., Ltd., Shanghai 200233 (CN)
(72) Inventor: LI, Zhenyan, Shanghai 200233 (CN); DONG, Shihua, Shanghai 200233 (CN); XU, Peng, Shanghai 200233 (CN)
(74) Representative: KIPA AB
(86) International application number: PCT/CN2021/109735
(87) International publication number: WO 2022/033331

(57) **Abstract**

A method for detecting nucleic acid comprises the following steps: S1, performing isothermal amplification of nucleic acid to be detected; S2, performing secondary amplification of the product of step S1, and the product of step S2 is a nucleic acid single strand which can be alternatively produced by other ways; S3, hybridizing the product of step S2 with a probe; S4, degrading probe: a nuclease is added into the hybridized product to degrade redundant single-strand nucleic acid; and, S5, detecting the product obtained in step S4. A kit for detecting nucleic acid comprises an isothermal amplification primer, a secondary amplification enzyme, a probe, a nuclease and a test strip. A use thereof comprises detection of pathogenic bacteria, viruses, transgenic nucleic acid, genetic material variation, tumor and DNA methylation variation. The aforementioned technical solution is rapid, specific and sensitive without a requirement of complex instruments and equipment.

## Description

### CROSS REFERENCE TO RELATED APPLICATION(S)

This patent application is a national phase of Ser. No.: PCT/CN2021/109735 filed on July 30, 2021, the entire content of which is incorporated by reference herein. This patent application claims the benefit and priority of Chinese Patent Application No. 202010803405.4 filed on August 8, 2020, the disclosure of which is incorporated by reference herein in its entirety as part of the present application.

### TECHNICAL FIELD

The present invention relates to the field of biotechnology, and in particular to a kit for detecting nucleic acid, a method for detecting nucleic acid and uses thereof.

### BACKGROUND

Nucleic acid detection is a method to detect the presence of target nucleic acid substances in samples such as viruses, bacteria, and cells. The nucleic acid detection method in the prior art mainly has three types: (1) conventional real-time quantitative fluorescence PCR detection which is the most common method at present: designing one or more pairs of primers for the sequence of the novel coronavirus, extracting nucleic acid, and performing reverse transcription and qPCR with a detection result which is the number of cycles; (2) on-site detection method that do not rely on large-scale instruments, such as DETECTER method based on the CRISPR/Cas12 developed by American researchers: the sample used in this method is the nucleic acid extracted from nasal swabs or throat swabs, which cannot meet the needs of patients for self-sampling and self-operation; and, (3) on-site or home-based detection that is a simple detection method in which the tester can self-sample, self-operate and self- read results.

Home-based detection does not rely on large-scale instruments, and is a rapid detecting method by self-sampling for the novel coronavirus, which will greatly improve the detection efficiency and safety. However, currently, there is still a lack of a rapid, specific, sensitive kit and method for detecting nucleic acid without the requirement of complex equipment.

*Salmonella* is a kind of common food-borne pathogenic bacteria. Accidental ingestion of food containing *Salmonella* can cause poisoning to humans, livestock and wild animals, and endanger their life and health. Traditional *Salmonella* identification method is mainly based on cultural characteristics, antigenic characteristics, morphological characteristics, physiological and biochemical characteristics, etc. The nucleic acid detection of *Salmonella* has the advantages of high sensitivity and good specificity.

African swine fever virus (ASFV) is a double-stranded DNA virus that can infect pigs of different ages and breeds. With extremely high infectivity, pathogenicity and mortality, the fatality rate of ASFV infection is extremely high which can be up to 100%, thereby ASFV infection has been classified as a first-class animal epidemic disease in China.

Novel coronavirus (SARS-CoV-2) is a RNA virus that can cause symptoms such as fever, cough, shortness of breath, and difficulty breathing in humans. In some more severe cases, SARS-CoV-2 infection can lead to pneumonia, severe acute respiratory syndrome, kidney failure, and even death. Traditional detection of SARS-CoV-2 is mainly carried out by laboratory real-time fluorescence quantitative PCR.

With the rapid development of transgenic technology, it has been widely used in the production of various animals and plants, such as rice, soybean, fish farming, cattle and sheep farming and so on. At the same time, the transgenic technology also brings some potential safety hazards. Illegal cultivation and sale of transgenic products such as rice, soybeans, beef and mutton occasionally occur. Risk assessment and risk management of transgenic products are an important part of the application of transgenic technology. Therefore, there is a great significance in accurate detection of transgenes products.

Detection of food composition is a detection of various foods, including pork, chicken, beef, canned meat, frozen meat, cured meat, imported meat, etc.

Genetic material variation refers to a phenomenon of changes in the genetic material of living organisms under physiological or pathological conditions, including mutation, recombination, deletion, chromosomal variation and other types. In the present disclosure, for the detection of BRAF gene deletion, a new method for detection of genetic material variation nucleic acid is provided. BRAF is a human gene that encodes the B-Raf protein. B-Raf protein participates in cell growth, involves a variety of signal transduction pathways, and affects processes such as cell division, differentiation and secretion. BRAF gene variation has been proved to be closely related to the occurrence of various tumors, and BRAFV 600E is the main mutation mode.

DNA methylation participates in various physiological and pathological processes, and is closely related to growth, differentiation, regeneration and diseases. The detection of DNA methylation variation can be used in tumor detection, prenatal diagnosis, technology research and development, disease diagnosis and other fields. In the present disclosure, for the methylation detection of Septin9 gene, a rapid methylation detection method is provided. Septin9 methylation variation has been widely reported to be closely related to the occurrence and development of a variety of tumors and can be used for the early detection of a variety of tumors.

### SUMMARY

The present application overcomes the defects of the prior art and provides a kit for detecting nucleic acid, a method for detecting nucleic acid and uses thereof, which are rapid, specific and sensitive without a requirement of complex instruments and equipment.

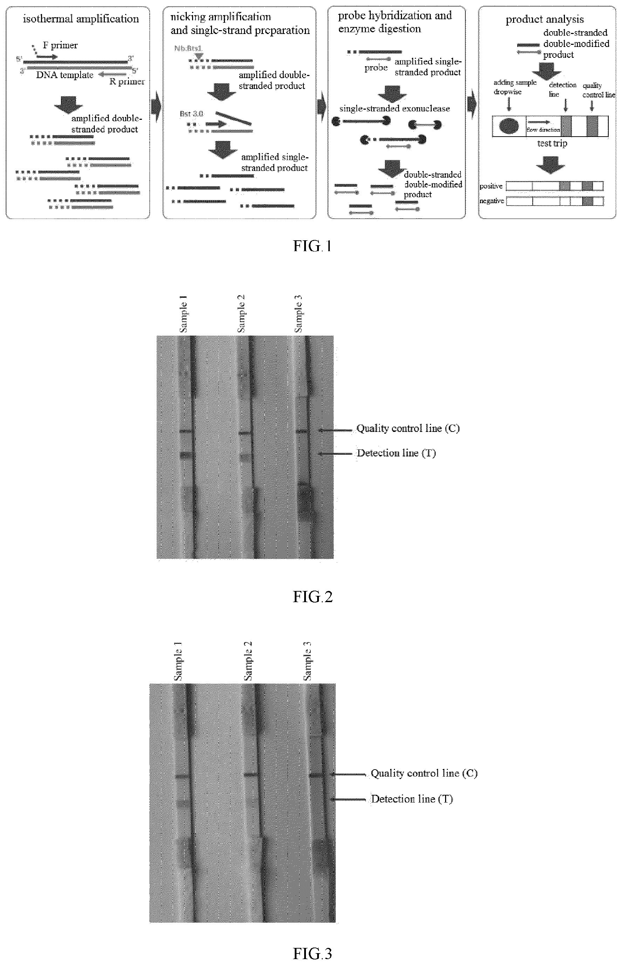

A first aspect of the present invention provides a method for detecting nucleic acid, as shown in FIG. 1, comprising the steps of:
S1, performing isothermal amplification: nucleic acid to be detected is subjected to an isothermal amplification;
S2, performing secondary amplification: the product of the isothermal amplification is subjected to a secondary amplification, and the product of the secondary amplification is a nucleic acid single strand; alternatively, Step S2 can be replaced by other ways to generate single strands, such as in vitro transcription, T7 exonuclease treatment, etc.
S3, hybridizing with probe: the product of the secondary amplification is hybridized with a probe;
S4, degrading probe: a nuclease is added into the hybridized product to degrade redundant single-strand nucleic acid; and,
S5, detecting product: the product obtained in step S4 is detected.

Preferably, the method for the isothermal amplification in step S1 and the secondary amplification in step S2 comprises at least one of recombinase polymerase amplification, recombinase-aided amplification, loop-mediated isothermal amplification, strand displacement isothermal amplification, rolling circle amplification, nucleic acid sequence-based amplification, helicase-dependent isothermal DNA amplification and nicking endonuclease amplification reaction.

Recombinase polymerase amplification (RPA): RPA mainly relies on the recombinase, single-stranded DNA binding protein (SSB) and strand displacing DNA polymerase that can bind to single-stranded nucleic acid (oligonucleotide primer). A protein-DNA complex can be formed by the combination of recombinase and primer, which can be used to search for homologous sequences in double-stranded DNA. Once the homologous sequence is located by the primer, a strand displacing reaction will be performing to initiate DNA synthesis, exponentially amplifying the target region on the template. Replaced DNA strand binds to the SSB, preventing further replacement. In this reaction system, a synthesis is initiated by two relative primers. The whole process can be carried out at room temperature, and generally the amplification product with a detectable level can be obtained in about 10 minutes.

Recombinase-aided amplification (RAA): RAA is a method that can rapidly amplify nucleic acid at room temperature. Recombinase used in RAA can be obtained from bacteria or fungi. The recombinase can be tightly combined with a primer to form a polymer. When the primer is paired with the template, with the help of single-stranded DNA binding protein, the template DNA can be unstranded, and a new DNA complementary strand can be formed under the action of DNA polymerase.

Loop-mediated isothermal amplification (LAMP): LAMP uses strand-displacement DNA polymerase to amplify under constant temperature conditions, in which 4-6 primers are designed for the region to be detected on the target gene. The amplification reaction can be carried out within 15-60 minutes under the temperature of 60-65 °C, and a large amount of amplification products can be produced during the amplification reaction.

Strand displacement isothermal amplification (SDA): SDA is based on nicking endonuclease. It is simpler, faster, more environmentally friendly and less expensive than traditional methods. It has very strong practical application potential and is especially suitable for on-site detection.

Rolling circle amplification (RCA): RCA uses a circular DNA as a template to synthesize a new circular DNA molecule. A nick is created at the origin of replication of a single strand, and then a new single strand is continuously synthesized using the other single strand as a template. The released newly synthesized single-stranded DNA is firstly copied into double-stranded DNA and is secondly cut into a strand with a unit length by an enzyme, and then a circular double-stranded DNA molecule is formed. Or, the released newly synthesized single-stranded DNA is firstly cut into a strand with a unit length by the enzyme to form a single-stranded circular DNA molecule, and then is replicated to form a double-stranded circular DNA molecule.

Nucleic acid sequence-based amplification (NASBA): NASBA is a continuous and uniform enzymatic process, which is a specifically isothermal amplification of nucleotide sequences in vitro. The enzymatic process is mediated by two primers using RNA in the nucleic acid sequence as a template. The whole reaction consists of an acyclic phase and a cyclic phase. Firstly, the acyclic phase is carried out. Under the action of AMV reverse transcriptase, primer 1 and template RNA are annealed to synthesize cDNA to form RNA/DNA hybrid, and then RNA is degraded by RNaseH; primer 2 and cDNA are annealed to synthesize a second complementary DNA strand. A promoter sequence of the formed double-stranded DNA is recognized by T7 RNA polymerase, and then can be catalyzed to synthesize RNA, entering the cyclic phase and amplifying the template in large quantities.

Helicase-dependent isothermal DNA amplification (HDA): HDA simulates a natural process of DNA replication in vivo. Helicase is used to unwind double-stranded DNA at a constant temperature. A single-stranded DNA after unwinding is stabilized by a single-stranded DNA binding protein and is provided as a template for primer, and then a complementary double-stranded DNA is synthesized under the action of DNA polymerase. The above-mentioned cyclic amplification process is repeated continuously, and finally the exponential growth of the target sequence is realized.

Nicking endonuclease amplification reaction (NEAR): NEAR is a strand displacement amplification technology. A nick is formed under the nicking of a nucleic acid nicking endonuclease. The raw material dNTPs is polymerized and extended from the 3' end of the nick to replace the allelic DNA strand under action of polymerase, thereby forming a new and complete DNA sequence containing a nickase recognition site. This double-stranded DNA is recognized and nicked by the nucleic acid nicking endonuclease again, and then starts the cycle of "polymerization-nicking", resulting in a large number of displaced DNA single strands in an exponential amplification.

Preferably, the method for the isothermal amplification in step S1 is recombinase polymerase amplification, and the method for the secondary amplification in step S2 is nicking endonuclease amplification reaction.

A nicking endonuclease is required to be added in the nicking endonuclease amplification reaction to react with a nucleic acid polymerase with a strand displacement function. The single strand can be nicked by the nicking endonuclease according to the site of primer, thereby forming a nick. The nucleic acid polymerase with a strand displacement function will start to amplify from the nicked position and perform a linear amplification of the unnicked strand as a template, and then a large number of single-stranded nucleic acid products can be obtained.

Preferably, a nicking endonuclease used in the nicking endonuclease amplification reaction comprises at least one of Nb.BtsI, Nt.BspQI, Nt.CviPII, Nt.BstNBI, Nb.BsrDI, Nb.BtsI, Nt.AlwI,

Nb.BbvCI, Nt.BbvCI, Nb.BsmI, Nb.BssSI and Nt.BsmAI; and, a strand displacement nucleic acid polymerase used in the nicking endonuclease amplification reaction comprises at least one of Bst3.0 DNA polymerase, Bst2.0 DNA polymerase, Klenow DNA polymerase and phi29 DNA polymerase. More preferably, the nicking endonuclease used in the nicking endonuclease amplification reaction is Nb.BtsI, the strand displacement nucleic acid polymerase used in the nicking endonuclease amplification reaction is Bst3.0 DNA polymerase.

Preferably, the probe used in step S3 is a modified oligonucleotide probe. More preferably, the modification type of the probe comprises at least one of biotin modification, digoxigenin modification, fluorescein isothiocyanate modification (FITC), Texas red modification (Texas Red-X), phosphorylation modification, amino modification (AminolinkerC6/7/12), sulfhydryl modification (Thiol C6/Thiol-C6 S-S), thio modification (Phosphorothioate), fluorescent group modification (FAM/HEX/ROX/CY-3/CY-5/JOE/TET, etc.) and quencher group modification (MGB/BHQ1/BHQ2/TAMRA, etc.). Most preferably, the modification type of the probe comprises digoxigenin modification and biotin modification.

Preferably, the nuclease used in step S4 comprises at least one of exonuclease VII, λ exonuclease, T5 exonuclease, T7 exonuclease, exonuclease V, exonuclease III, RecJ1 exonuclease and exonuclease I. More preferably, the exonuclease used is exonuclease VII.

Preferably, a product detecting method in step S5 comprises at least one of colloidal gold test strip method, colloidal carbon test strip method, fluorescent dye method, ultraviolet irradiation method, and real-time fluorescence quantitative PCR method. More preferably, the product detecting method is colloidal gold test strip method.

Colloidal gold test strip method: colloidal gold is a commonly used labeling material. It is a new type of immunolabeling technology applied to antigen and antibody using colloidal gold as a tracer marker. It has its unique advantages. It has been widely used in various biological researches in recent years. At the same time, it may be used in flow, electron microscopy, immunity, nucleic acid detection, molecular biology and even biochips.

Colloidal carbon test strip method: according to the principle of colloidal gold test strip, the gold particles are replaced with carbon particles, which is more sensitive.

Fluorescent dye method: fluorescent dye refers to a substance that absorbs a light wave of a certain wavelength and emits another light wave with a wavelength greater than that of the absorbed light. Most of the fluorescent dyes are compounds containing a benzene ring or a heterocyclic ring with a conjugated double bond. Fluorescent dyes can be used alone or can be combining used as composite fluorescent dyes. It can be used in molecular biology experiments such as nucleic acid detection.

Ultraviolet irradiation method: after adding some specific nucleic acid dyes, such as ethidium bromide, SYBR, etc., the amplified nucleic acid can be detected by ultraviolet light irradiation.

Real-time fluorescence quantitative PCR method: the amplified nucleic acid can be detected by an instrument, such as a real-time quantitative PCR (qPCR) instrument.

Preferably, the nucleic acid to be detected in the step S1 comprises at least one of DNA nucleic acid samples, RNA nucleic acid samples and DNA nucleic acid samples reversely transcribed by RNA.

A second aspect of the present invention provides a use of the method according to any one of the first aspect of present invention in detecting pathogenic bacteria, viruses, transgenic nucleic acid, food components, genetic material variation and DNA methylation variation.

Preferably, for detecting pathogenic bacteria and viruses, the material to be detected comprises *Salmonella,* African swine fever virus and novel coronavirus.

Preferably, for detecting transgenic nucleic acid, food components, genetic material variation and DNA methylation variation, the material to be detected comprises NOS gene, porcine mitochondrial CYTB gene, BRAF gene and Septin9 gene, respectively.

Preferably, for detecting *Salmonella,* the nucleotide sequence of the isothermal amplification primer(s) in step S1 is SEQ ID NO.1 and SEQ ID NO.2.

Preferably, for detecting *Salmonella,* the nucleotide sequence of the probe in step S3 is SEQ ID NO.3

Preferably, for detecting African swine fever virus, the nucleotide sequence of the isothermal amplification primer(s) in step S1 is SEQ ID NO.4 and SEQ ID NO.5.

Preferably, for detecting African swine fever virus, the nucleotide sequence of the probe in step S3 is SEQ ID NO.6.

Preferably, for detecting NOS gene, the nucleotide sequence of the isothermal amplification primer(s) in step S1 is SEQ ID NO.7 and SEQ ID NO.8.

Preferably, for detecting NOS gene, the nucleotide sequence of the probe in step S3 is SEQ ID NO.9.

Preferably, for detecting porcine mitochondrial CYTB gene, the nucleotide sequence of the isothermal amplification primer(s) in step S1 is SEQ ID NO.10 and SEQ ID NO.11.

Preferably, for detecting porcine mitochondrial CYTB gene, the nucleotide sequence of the probe in step S3 is SEQ ID NO.12.

Preferably, for detecting BRAF gene, the nucleotide sequence of the isothermal amplification primer(s) in step S1 is SEQ ID NO. 13 and SEQ ID NO. 14.

Preferably, for detecting BRAF gene, the nucleotide sequence of the probe in step S3 is SEQ ID NO.15.

Preferably, for detecting Septin9 gene, the nucleotide sequence of the isothermal amplification primer(s) in step S1 is SEQ ID NO. 16 and SEQ ID NO. 17.

Preferably, for detecting Septin9 gene, the nucleotide sequence of the probe in step S3 is SEQ ID NO.18.

Preferably, for detecting novel coronavirus, the nucleotide sequence of the isothermal amplification primer(s) in step S1 is SEQ ID NO.20 and SEQ ID NO.21.

Preferably, for detecting novel coronavirus, the nucleotide sequence of the probe in step S3 is SEQ ID NO.19.

The above nucleic acid sequences are shown in Table 1.

**Table 1**

| ID No. | Sequence |
|---|---|
| SEQ ID NO.1 | CCTACAACTTGTGCTAATGCACTGCCGCTTCCAGTTGGTCCAGCATATGTTTTGT TTC |
| SEQ ID NO.2 | GCTTGATGAGCTTTACCACTGTCTGGCGGTGAC |
| SEQ ID NO.3 | 5'DIGOXIGENIN-TGGCACGCAACGTCAATGAATATTTCGGTATTCAG-3'BIOTIN |
| SEQ ID NO.4 | CCTACAACTTGTGCTAATGCACTGCAGCTCTTACATACCCTTCCACTACGGAGG |
| | CAGT |
| SEQ ID NO.5 | ACTGGGTTGATATTCCTCCCGTGGCTTCAAAGC |
| SEQ ID NO.6 | 5'DIGOXIGENIN-CATCATCGCACCAGGATCGTCAGGGGTTTTAATCG-3'BIOTIN |
| SEQ ID NO.7 | CCTACAACTTGTGCTAATGCACTGGGTGATAACTCCTTCACTGGCCTTGATTGCT GA |
| SEQ ID NO.8 | AGCGGCTTGGTTCGCCTCCCGGCACGTTCCCAC |
| SEQ ID NO.9 | 5'DIGOXIGENIN-CGCTGTGGGCACCAGCAGATCATCCAGGATCGATC-3'BIOTIN |
| SEQ ID NO.10 | CCTACAACTTGTGCTAATGCACTGCACCGTTATAGCAACAGCCTTCATAGGCTA CGTC |
| SEQ ID NO.11 | TAAGGGATAGCTGATAGTAGATTTGTGATGACC |
| SEQ ID NO.12 | 5'DIGOXIGENIN-GTAGCTCCTCAGAATGATATTTGTCCTCAGGGCAG-3'BIOTIN |
| SEQ ID NO.13 | CCTACAACTTGTGCTAATGCACTGCATATTTCTTCATGAAGACCTCACAGTAAAA ATAG |
| SEQ ID NO.14 | CCACAAAATGGATCCAGACAACTGTTCAAACTGA |
| SEQ ID NO.15 | 5'DIGOXIGENIN-GACCCACTCCATCGAGAAGCTAGACCAAAAT-3'BIOTIN |
| SEQ ID NO.16 | CCTACAACTTGTGCTAATGCACTGCCCCCGCCGGGGGCGCTTCCTGCCGCTGC CTCCG |
| SEQ ID NO.17 | CCTCTCCAGCACGTCCGCGGCCGCAGCAGCCAGC |
| SEQ ID NO.18 | 5'DIGOXIGENIN-AGCTGCGCGTTGACCGCGGGGTCCGACATGATG-3'BIOTIN |
| SEQ ID NO.19 | 5'DIGOXIGENIN-CAGCCATAACCTTTCCACATACCGCAGACG-3'BIOTIN |
| SEQ ID NO.20 | CCTACAACTTGTGCTAATGCACTGCCCTACAACTTGTGCTAATGACCCTGTGGG TTTTAC |
| SEQ ID NO.21 | CCCGTTTAAAAACGATTGTGCATCAGCTGAC |

A third aspect of the present invention provides a kit for detecting nucleic acid comprising an isothermal amplification primer, a secondary amplification enzyme, a probe, and a nuclease.

Preferably, the probe is a modified oligonucleotide probe. More preferably, the modification type of the probe comprises at least one of biotin modification, digoxigenin modification, fluorescein isothiocyanate modification (FITC), Texas red modification (Texas Red-X), phosphorylation modification, amino modification (AminolinkerC6/7/12), sulfhydryl modification (Thiol C6/Thiol-C6 S-S), thio modification (Phosphorothioate), fluorescent group modification (FAM/HEX/ROX/CY-3/CY-5/JOE/TET, etc.) and quencher group modification (MGB/BHQ1/BHQ2/TAMRA, etc.). Most preferably, the modification type of the probe comprises digoxigenin modification and biotin modification.

Preferably, the nucleotide sequence of the probe comprises SEQ ID NO.3, SEQ ID NO.6, SEQ ID NO.9, SEQ ID NO.12, SEQ ID NO. 15, SEQ ID NO.18, and SEQ ID NO.19.

Preferably, the nucleotide sequence of the isothermal amplification primer comprises SEQ ID NO.1, SEQ ID NO.2, SEQ ID NO.4, SEQ ID NO.5, SEQ ID NO.7, SEQ ID NO.8, SEQ ID NO.10, SEQ ID NO.11, SEQ ID NO 13, SEQ ID NO.14, SEQ ID NO.16, SEQ ID NO. 17, SEQ ID NO.11 ID NO.20, and SEQ ID NO.21.

Preferably, the secondary amplification enzyme comprises at least one of a nicking endonuclease and a strand displacement nucleic acid polymerase, in which the nicking endonuclease comprises at least one of Nb.BtsI, Nt.BspQI, Nt.CviPII, Nt.BstNBI, Nb.BsrDI,

Nb.BtsI, Nt.AlwI, Nb.BbvCI, Nt.BbvCI, Nb.BsmI, Nb.BssSI and Nt.BsmAI; and, the strand displacement nucleic acid polymerase comprises at least one of Bst3.0 DNA polymerase, Bst2.0 DNA polymerase, Klenow DNA polymerase and phi29 DNA polymerase.

Preferably, the nuclease comprises at least one of exonuclease VII, λ exonuclease, T5 exonuclease, T7 exonuclease, exonuclease V, exonuclease III, RecJ1 exonuclease and exonuclease I.

Preferably, the kit further comprises a test strip which comprises a colloidal gold test strip or a colloidal carbon test strip.

The present invention has the following advantages over the prior art: a new nucleic acid rapid detection method is provided, which does not need to rely on complex laboratory equipment, and can complete nucleic acid detection in the wilderness field, at home, at the detection site, etc.. The method has the advantages of simple operation, high specificity, no dependence on complex instruments and so on. Using this method, various nucleic acid detection, such as pathogenic microorganism nucleic acid detection, viral nucleic acid detection, transgenic nucleic acid detection, genetic disease nucleic acid detection, tumor nucleic acid detection, and DNA methylation nucleic acid detection, can be performed.

### BRIEF DESCRIPTION OF THE DRAWINGS

The accompanying drawings described here are provided for further understanding of the present disclosure, and constitute a part of the present disclosure. The exemplary embodiments of the present disclosure and illustrations thereof are intended to explain the present disclosure, but do not constitute inappropriate limitations to the present disclosure. In the drawings:
FIG. 1 is a principle schematic diagram of a method of the present invention;
FIG. 2 is a nucleic acid detection result of *Salmonella* according to embodiment 1 of the present invention;
FIG. 3 is a nucleic acid detection result of African swine fever virus according to embodiment 2 of the present invention;
FIG. 4 is a nucleic acid detection result of NOS gene according to embodiment 3 of the present invention;
FIG. 5 is a nucleic acid detection result of porcine mitochondrial CYTB gene according to embodiment 4 of the present invention;
FIG. 6 is a nucleic acid detection result of BRAF gene according to embodiment 5 of the present invention;
FIG. 7 is a nucleic acid detection result of Septin9 gene according to embodiment 6 of the present invention;
FIG. 8 is a detection result of novel coronavirus (SARS-CoV-2) probe in a degradation test according to embodiment 7 of the present invention;
FIG. 9 is a detection result of using novel coronavirus (SARS-CoV-2) DNA standard and RNA standard according to embodiment 8 of the present invention;
FIG. 10 is a detection result of novel coronavirus (SARS-CoV-2) by using clinical throat swab samples according to embodiment 9 of the present invention;
FIG. 11 is a detection result of novel coronavirus (SARS-CoV-2) by using clinical mouthwash samples according to embodiment 10 of the present invention.

### DETAILED DESCRIPTION

To make the objectives, technical solutions, and advantages of the present disclosure clearer, the present disclosure is described below with reference to the accompanying drawings and embodiments. It should be understood that the embodiments described herein are merely used to explain the present disclosure, rather than to limit the present disclosure. All other embodiments obtained by those of ordinary skill in the art based on the embodiments of the present disclosure without creative efforts should fall within the protection scope of the present disclosure.

Apparently, the accompanying drawings in the following description show merely some embodiments of the present disclosure, and a person of ordinary skill in the art may apply the present disclosure to other similar scenarios according to these drawings without creative efforts. In addition, it can also be appreciated that, although it may take enduring and complex efforts to achieve such a development process, for those of ordinary skill in the art related to the present disclosure, some changes such as design, manufacturing or production made based on the technical content in the present disclosure are merely regular technical means, and should not be construed as insufficiency of the present disclosure.

The "embodiment" mentioned in the present disclosure means that a specific feature, structure, or characteristic described in combination with the embodiment may be included in at least one embodiment of the present disclosure. The phrase appearing in different parts of the specification does not necessarily refer to the same embodiment or an independent or alternative embodiment exclusive of other embodiments. It may be explicitly or implicitly appreciated by those of ordinary skill in the art that the embodiment described herein may be combined with other embodiments as long as no conflict occurs.

Unless otherwise defined, the technical or scientific terms used in the present disclosure are as they are usually understood by those of ordinary skill in the art to which the present disclosure pertains. The terms "one", "a", "the" and similar words are not meant to be limiting, and may represent a singular form or a plural form. The terms "comprise", "include", "contain", "have" and any other variants in the present disclosure mean to cover the non-exclusive inclusion, for example, a process, method, system, product, or device that includes a series of steps or modules (units) is not necessarily limited to those steps or units which are clearly listed, but may include other steps or units which are not expressly listed or inherent to such a process, method, system, product, or device. The term "and/or" describes associations between associated objects, and it indicates three types of relationships. For example, "A and/or B" may indicate that A exists alone, A and B coexist, or B exists alone. The character "/" generally indicates that the associated objects are in an "or" relationship.

The experimental methods that do not specify specific conditions in the following examples are selected according to conventional methods and conditions in the field, or according to commercial instructions. The relevant reagents and biological materials in the following examples are all commercially available products. The molecules cloning technology in the following examples is a means of purifying and amplifying specific DNA fragments at the molecular level in the prior art.

The following examples mainly describe several nucleic acid detection, such as *Salmonella* detection, African swine fever virus detection, transgenic detection, food components detection, genetic variation detection, DNA methylation detection and novel coronavirus detection.

### Embodiment 1 - nucleic acid detecting of Salmonella

The method for detecting nucleic acid of *Salmonella* mainly comprises the following steps:
1.1 - Preparation of test samples: use a bacterial nucleic acid extraction kit to extract the nucleic acid of *Salmonella,* and then prepare the following three samples: positive sample 1 containing *Salmonella* nucleic acid fragments (sample 1), positive sample 2 containing *Salmonella* nucleic acid fragments (sample 2), and negative sample 3 that do not contain *Salmonella* nucleic acid fragments (sample 3).
1.2 - Design of primers and probes: isothermal amplification primers and detection probes are designed based on the extracted *Salmonella* genome sequence. The amplification forward primer sequence is SEQ ID NO. 1, the amplification reverse primer sequence is SEQ ID NO. 2, and the detection probe sequence is SEQ ID NO. 3.
1.3 - Isothermal amplification: RNA isothermal rapid amplification kit (item No.: WLRN8206KIT) produced by Weifang Anpu Future Corp. is used for amplification. The kit reagents contain a reverse transcriptase, which can be used to amplify DNA templates or RNA templates. The isothermal amplification reaction system and conditions are as follows:
Reaction system:

| Reagents | Volume |
|---|---|
| powder | 1 tube |
| Buffer A | 29.4 µL |
| SEQ ID NO.1 (10 µM) | 0.5 µL |
| SEQ ID NO.2 (10 µM) | 0.5 µL |
| Template (positive sample/ negative sample/NTC) | 17.1 µL |
| Buffer B | 2.5 µL |
| Overall system | 50 µL |

Reaction conditions: 37°C for 15 minutes.
1.4 - Secondary amplification: in the above reaction system, add 1 µL Bst3.0 (8U), 1µL Nb.BtsI (10U), 2 µL dNTPs (2.5 mM each), mix well, react at 37°C for 15 minutes, and react at 95°C for 5 minutes.
1.5 - Probe hybridization: add 2 µL (10 µM) labeled probe into the above system, place the system in a boiling water cup for 1 min, and slowly cool down the system together with the cup (about 15 minutes), take out 20 µL reaction system, add 0.5 µL exonuclease VII into the 20 µL reaction system, and react at 37°C for 5 minutes.
1.6 - Colloidal gold test strip detection: add 60 µL ddH₂O in the reaction system, mix well, add sample dropwise on a sample hole of the colloidal gold test strip, and observe the change of the test line and the quality control line within 5 minutes.
1.7 - Results: as shown in FIG. 2, all samples have bands at the position of the quality control line (C), indicating that the experimental results are valid. Sample 1 has a band at the position of the detection line (T), which is positive; sample 2 has a band at the detection line (T) position, which is positive; and, sample 3 has no band at the detection line (T) position, which is negative. It is indicated that the method provided by the present invention can accurately detect Salmonella.

### Embodiment 2 - nucleic acid detecting of African swine fever virus

The method for detecting nucleic acid of African swine fever virus mainly comprises the following steps:
2.1 - Preparation of test samples: use a viral nucleic acid extraction kit to extract the nucleic acid of African swine fever virus, and then prepare the following three samples: positive sample 1 containing African swine fever virus nucleic acid fragments (sample 1), positive sample 2 containing African swine fever virus nucleic acid fragments (sample 2), and negative sample 3 that do not contain African swine fever virus nucleic acid fragments (sample 3).
2.2 - Design of primers and probes: isothermal amplification primers and detection probes are designed based on the extracted African swine fever virus genome sequence. The amplification forward primer sequence is SEQ ID NO. 4, the amplification reverse primer sequence is SEQ ID NO. 5, and the detection probe sequence is SEQ ID NO. 6.
2.3 - Isothermal amplification: RNA isothermal rapid amplification kit (item No.: WLRN8206KIT) produced by Weifang Anpu Future Corp. is used for amplification. The kit reagents contain a reverse transcriptase, which can be used to amplify DNA templates or RNA template. The isothermal amplification reaction system and conditions are as follows:
Reaction system:

| Reagents | Volume |
|---|---|
| powder | 1 tube |
| Buffer A | 29.4 µL |
| SEQ ID NO.4 (10 µM) | 0.5 µL |
| SEQ ID NO.5 (10 µM) | 0.5 µL |
| Template (positive sample/ negative sample/NTC) | 17.1 µL |
| Buffer B | 2.5 µL |
| Overall system | 50 µL |

Reaction conditions: 37°C for 15 minutes.
2.4 - Secondary amplification: in the above reaction system, add 1 µL Bst3.0 (8U), 1µL Nb.BtsI (10U), 2 µL dNTPs (2.5 mM each), mix well, react at 37°C for 15 minutes, and react at 95°C for 5 minutes.
2.5 - Probe hybridization: add 2 µL (10 µM) labeled probe into the above system, place the system in a boiling water cup for 1 min, and slowly cool down the system together with the cup (about 15 minutes), take out 20 µL reaction system, add 0.5 µL exonuclease VII into the 20 µL reaction system, and react at 37°C for 5 minutes.
2.6 - Colloidal gold test strip detection: add 60 µL ddH₂O in the reaction system, mix well, add sample dropwise on a sample hole of the colloidal gold test strip, and observe the change of the test line and the quality control line within 5 minutes.
2.7 - Results: as shown in FIG. 3, all samples have bands at the position of the quality control line (C), indicating that the experimental results are valid. Sample 1 has a band at the position of the detection line (T), which is positive; sample 2 has a band at the detection line (T) position, which is positive; and, sample 3 has no band at the detection line (T) position, which is negative. It is indicated that the method provided by the present invention can accurately detect African swine fever virus.

### Embodiment 3 - nucleic acid detecting of transgenosis

The method for detecting transgenic nucleic acid takes the detection of NOS gene as an example. The method for detecting NOS gene mainly comprises the following steps:
3.1 - Preparation of test samples: prepare the following three samples: positive sample 1 containing NOS gene nucleic acid fragments (sample 1), positive sample 2 containing NOS gene nucleic acid fragments (sample 2), and negative sample 3 that do not contain NOS gene nucleic acid fragments (sample 3).
3.2 - Design of primers and probes: isothermal amplification primers and detection probes are designed based on the extracted NOS gene nucleic acid sequence. The amplification forward primer sequence is SEQ ID NO. 7, the amplification reverse primer sequence is SEQ ID NO. 8, and the detection probe sequence is SEQ ID NO. 9.
3.3 - Isothermal amplification: RNA isothermal rapid amplification kit (item No.: WLRN8206KIT) produced by Weifang Anpu Future Corp. is used for amplification. The kit reagents contain a reverse transcriptase, which can be used to amplify DNA templates or RNA template. The isothermal amplification reaction system and conditions are as follows:
Reaction system:

| Reagents | Volume |
|---|---|
| powder | 1 tube |
| Buffer A | 29.4 µL |
| SEQ ID NO. 7 (10 µM) | 0.5 µL |
| SEQ ID NO. 8 (10 µM) | 0.5 µL |
| Template (positive sample/ negative sample/NTC) | 17.1 µL |
| Buffer B | 2.5 µL |
| Overall system | 50 µL |

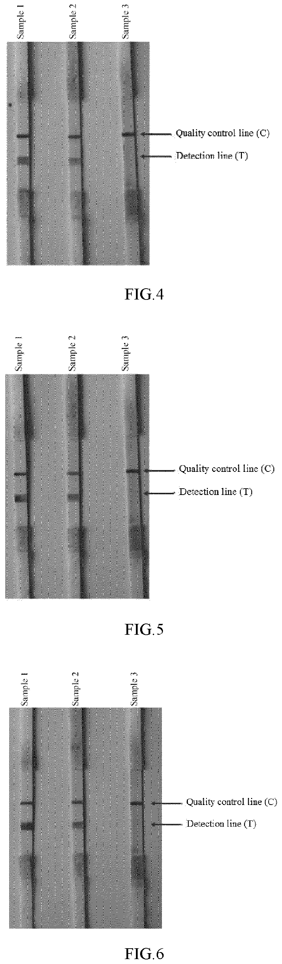

Reaction conditions: 37°C for 15 minutes.
3.4 - Secondary amplification: in the above reaction system, add 1 µL Bst3.0 (8U), 1µL Nb.BtsI (10U), 2 µL dNTPs (2.5 mM each), mix well, react at 37°C for 15 minutes, and react at 95°C for 5 minutes.
3.5 - Probe hybridization: add 2 µL (10 µM) labeled probe into the above system, place the system in a boiling water cup for 1 min, and slowly cool down the system together with the cup (about 15 minutes), take out 20 µL reaction system, add 0.5 µL exonuclease VII into the 20 µL reaction system, and react at 37°C for 5 minutes.
3.6 - Colloidal gold test strip detection: add 60 µL ddH₂O in the reaction system, mix well, add sample dropwise on a sample hole of the colloidal gold test strip, and observe the change of the test line and the quality control line within 5 minutes.
3.7 - Results: as shown in FIG. 4, all samples have bands at the position of the quality control line (C), indicating that the experimental results are valid. Sample 1 has a band at the position of the detection line (T), which is positive; sample 2 has a band at the detection line (T) position, which is positive; and, sample 3 has no band at the detection line (T) position, which is negative. It is indicated that the method provided by the present invention can accurately detect NOS gene.

### Embodiment 4 - nucleic acid detecting of food components

The method for detecting food components takes the detection of pork components as an example. The method for detecting porcine mitochondrial CYTB gene mainly comprises the following steps:
4.1 - Preparation of test samples: prepare the following three samples: positive sample 1 containing porcine mitochondrial CYTB gene nucleic acid fragments (sample 1), positive sample 2 containing porcine mitochondrial CYTB gene nucleic acid fragments (sample 2), and negative sample 3 that do not contain porcine mitochondrial CYTB gene nucleic acid fragments (sample 3).
4.2 - Design of primers and probes: isothermal amplification primers and detection probes are designed based on the extracted porcine mitochondrial CYTB gene nucleic acid sequence. The amplification forward primer sequence is SEQ ID NO. 10, the amplification reverse primer sequence is SEQ ID NO. 11, and the detection probe sequence is SEQ ID NO. 12.
4.3 - Isothermal amplification: RNA isothermal rapid amplification kit (item No.: WLRN8206KIT) produced by Weifang Anpu Future Corp. is used for amplification. The kit reagents contain a reverse transcriptase, which can be used to amplify DNA templates or RNA template. The isothermal amplification reaction system and conditions are as follows:
Reaction system:

| Reagents | Volume |
|---|---|
| powder | 1 tube |
| Buffer A | 29.4 µL |
| SEQ ID NO. 10 (10 µM) | 0.5 µL |
| SEQ ID NO. 11 (10 µM) | 0.5 µL |
| Template (positive sample/ negative sample/NTC) | 17.1 µL |
| Buffer B | 2.5 µL |
| Overall system | 50 µL |

Reaction conditions: 37°C for 15 minutes.
4.4 - Secondary amplification: in the above reaction system, add 1 µL Bst3.0 (8U), 1µL Nb.BtsI (10U), 2 µL dNTPs (2.5 mM each), mix well, react at 37°C for 15 minutes, and react at 95°C for 5 minutes.
4.5 - Probe hybridization: add 2 µL (10 µM) labeled probe into the above system, place the system in a boiling water cup for 1 min, and slowly cool down the system together with the cup (about 15 minutes), take out 20 µL reaction system, add 0.5 µL exonuclease VII into the 20 µL reaction system, and react at 37°C for 5 minutes.
4.6 - Colloidal gold test strip detection: add 60 µL ddH₂O in the reaction system, mix well, add sample dropwise on a sample hole of the colloidal gold test strip, and observe the change of the test line and the quality control line within 5 minutes.
4.7 - Results: as shown in FIG. 5, all samples have bands at the position of the quality control line (C), indicating that the experimental results are valid. Sample 1 has a band at the position of the detection line (T), which is positive; sample 2 has a band at the detection line (T) position, which is positive; and, sample 3 has no band at the detection line (T) position, which is negative. It is indicated that the method provided by the present invention can accurately detect porcine mitochondrial CYTB gene, thereby having a potential in food composition detection.

### Embodiment 5 - nucleic acid detecting of genetic material variation

The method for detecting genetic material variation takes the detection of BRAF gene as an example. The method for detecting BRAF gene mainly comprises the following steps:
5.1 - Preparation of test samples: prepare the following three samples: positive sample 1 containing BRAF gene variation nucleic acid fragments (sample 1), positive sample 2 containing BRAF gene variation nucleic acid fragments (sample 2), and negative sample 3 that do not contain BRAF gene variation nucleic acid fragments (sample 3).
5.2 - Design of primers and probes: isothermal amplification primers and detection probes are designed based on the extracted BRAF gene nucleic acid sequence. The amplification forward primer sequence is SEQ ID NO. 13, the amplification reverse primer sequence is SEQ ID NO. 14, and the detection probe sequence is SEQ ID NO. 15.
5.3 - Isothermal amplification: RNA isothermal rapid amplification kit (item No.: WLRN8206KIT) produced by Weifang Anpu Future Corp. is used for amplification. The kit reagents contain a reverse transcriptase, which can be used to amplify DNA templates or RNA template. The isothermal amplification reaction system and conditions are as follows:
Reaction system:

| Reagents | Volume |
|---|---|
| powder | 1 tube |
| Buffer A | 29.4 µL |
| SEQ ID NO. 13 (10 µM) | 0.5 µL |
| SEQ ID NO. 14 (10 µM) | 0.5 µL |
| Template (positive sample/ negative sample/NTC) | 17.1 µL |

| | |
|---|---|
| Buffer B | 2.5 µL |
| Overall system | 50 µL |

Reaction conditions: 37°C for 15 minutes.
5.4 - Secondary amplification: in the above reaction system, add 1 µL Bst3.0 (8U), 1µL Nb.BtsI (10U), 2 µL dNTPs (2.5 mM each), mix well, react at 37°C for 15 minutes, and react at 95°C for 5 minutes.
5.5 - Probe hybridization: add 2 µL (10 µM) labeled probe into the above system, place the system in a boiling water cup for 1 min, and slowly cool down the system together with the cup (about 15 minutes), take out 20 µL reaction system, add 0.5 µL exonuclease VII into the 20 µL reaction system, and react at 37°C for 5 minutes.
5.6 - Colloidal gold test strip detection: add 60 µL ddH₂O in the reaction system, mix well, add sample dropwise on a sample hole of the colloidal gold test strip, and observe the change of the test line and the quality control line within 5 minutes.
5.7 - Results: as shown in FIG. 6, all samples have bands at the position of the quality control line (C), indicating that the experimental results are valid. Sample 1 has a band at the position of the detection line (T), which is positive; sample 2 has a band at the detection line (T) position, which is positive; and, sample 3 has no band at the detection line (T) position, which is negative. It is indicated that the method provided by the present invention can accurately detect BRAF gene, thereby having a potential in genetic material variation detection.

### Embodiment 6 - nucleic acid detecting of DNA methylation

The method for detecting DNA methylation takes the detection of Septin9 gene methylation as an example. The method for detecting Septin9 gene methylation mainly comprises the following steps:
6.1 - Preparation of test samples: prepare the following three samples: positive sample 1 containing Septin9 gene hypermethylation nucleic acid fragments (sample 1), positive sample 2 containing Septin9 gene hypermethylation nucleic acid fragments (sample 2), and negative sample 3 that do not contain Septin9 gene hypomethylation nucleic acid fragments (sample 3).
6.2 - Design of primers and probes: isothermal amplification primers and detection probes are designed based on the extracted Septin9 gene nucleic acid sequence. The amplification forward primer sequence is SEQ ID NO. 16, the amplification reverse primer sequence is SEQ ID NO. 17, and the detection probe sequence is SEQ ID NO. 18.
6.3 - Isothermal amplification: RNA isothermal rapid amplification kit (item No.: WLRN8206KIT) produced by Weifang Anpu Future Corp. is used for amplification. The kit reagents contain a reverse transcriptase, which can be used to amplify DNA templates or RNA template. The isothermal amplification reaction system and conditions are as follows:
Reaction system:

| Reagents | Volume |
|---|---|
| powder | 1 tube |
| Buffer A | 29.4 µL |
| SEQ ID NO. 16 (10 µM) | 0.5 µL |
| SEQ ID NO. 17 (10 µM) | 0.5 µL |
| Template (positive sample/ negative sample/NTC) | 17.1 µL |
| Buffer B | 2.5 µL |
| Overall system | 50 µL |

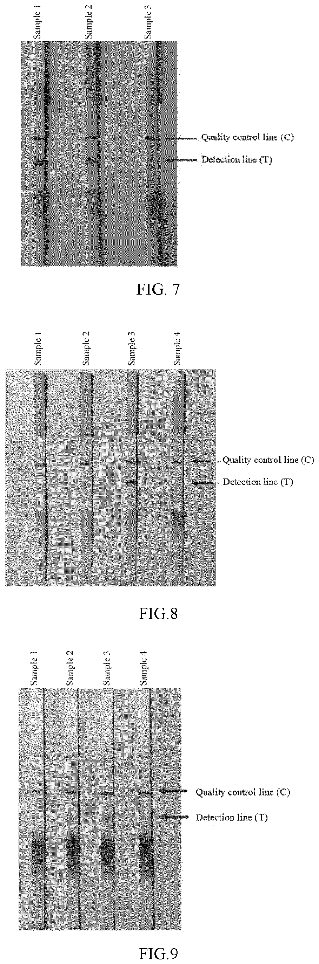

Reaction conditions: 37°Cfor 15 minutes.
6.4 - Secondary amplification: in the above reaction system, add 1 µL Bst3.0 (8U), 1µL Nb.BtsI (10U), 2 µL dNTPs (2.5 mM each), mix well, react at 37°C for 15 minutes, and react at 95°C for 5 minutes.
6.5 - Probe hybridization: add 2 µL (10 µM) labeled probe into the above system, place the system in a boiling water cup for 1 min, and slowly cool down the system together with the cup (about 15 minutes), take out 20 µL reaction system, add 0.5 µL exonuclease VII into the 20 µL reaction system, and react at 37°C for 5 minutes.
6.6 - Colloidal gold test strip detection: add 60 µL ddH₂O in the reaction system, mix well, add sample dropwise on a sample hole of the colloidal gold test strip, and observe the change of the test line and the quality control line within 5 minutes.
6.7 - Results: as shown in FIG. 7, all samples have bands at the position of the quality control line (C), indicating that the experimental results are valid. Sample 1 has a band at the position of the detection line (T), which is positive; sample 2 has a band at the detection line (T) position, which is positive; and, sample 3 has no band at the detection line (T) position, which is negative. It is indicated that the method provided by the present invention can accurately detect Septin9 gene, thereby having a potential in DNA methylation detection.

### Embodiment 7 - detection of novel coronavirus (SARS-CoV-2) probe in a degradation test

7.1 - Preparation of probe: design and modify a probe, the nucleic acid sequence of the probe is SEQ ID NO. 19, wherein the 5' end of the probe is labeled with digoxigenin modification, and the 3' end of the probe is labeled with biotin modification.
7.2 - Preparation of samples: a negative control group - water (sample 1), and experimental groups: 1 pmol modified probe (sample 2), 10 pmol modified probe (sample 3), and 10 pmol modified probe nicked by a single-strand exonuclease VII (sample 4).
7.3 - Colloidal gold test strip detection: take 50 µL product obtained in step 7.2, insert a colloidal gold test strip into the product and perform a static chromatography, and read the result within 5 minutes.
7.4 - Results: as shown in FIG. 8, all samples have bands at the position of the quality control line (C), indicating that the experimental results are valid. At the position of the detection line (T), sample 1 has no band, which is negative; sample 2 has a band, which is positive; sample 3 has a band, which is positive; and, sample 4 has no band, which is negative. It is indicated that the probe can be directly detected, and the probe nicked by exonuclease cannot be detected.

### Embodiment 8 - detection of novel coronavirus (SARS-CoV-2) DNA standard and RNA standard

8.1 - Preparation of test samples: a negative control group - water (sample 1), and experimental groups: 1000 copies of SARS-CoV-2 DNA fragments (sample 2), 10,000 copies of SARS-CoV-2 RNA standard (sample 3), and 1,000 copies of SARS-CoV-2 RNA standard (sample 4).

8.2 - First isothermal amplification: RNA isothermal rapid amplification kit (Anpu Future Corp., item No.: WLRN8206KIT) is used for amplification, based on the detecting samples obtained in step 8.1 as templates. The isothermal amplification steps refer to the specification of the kit.

Reaction system:

| Reagents | Volume |
|---|---|
| powder | 1 tube |
| Buffer A | 29.4 µL |
| SEQ ID NO. 20 (10 µM) | 0.5 µL |
| SEQ ID NO. 21 (10 µM) | 0.5 µL |
| Template (H₂O/DNA/RNA) | 17.1 µL |
| Buffer B | 2.5 µL |

Reaction conditions: 37°C for 12 minutes.

The amplification forward primer sequence is SEQ ID NO. 20, the amplification reverse primer sequence is SEQ ID NO. 21.

8.3 - Secondary amplification: in the above reaction system, add 1 µL Nb.BstI, 1µL Bts3.0, 4 µL dNTPs, react at 37°C for 15 minutes, and react at 95°C for 5 minutes.

8.4 - Probe hybridization: add 2 µL (10 µM) labeled probe into the above system, hybridize at 95°C for 1 minute, and slowly cool down to the room temperature (about 15 minutes).

8.5 - Degradation of redundant probe: take out 20 µL hybridized product, add 0.5 µL exonuclease VII into the 20 µL hybridized product, and react at 37°C for 5 minutes.

8.6 - Colloidal gold test strip detection: add 60 µL ddH₂O in the product obtained in step 8.5, mix well, insert a colloidal gold test strip into the product, and read the result within 5 minutes.

8.7 - Results: as shown in FIG. 9, all samples have bands at the position of the quality control line (C), indicating that the experimental results are valid. At the position of the detection line (T), sample 1 has no band, which is negative; sample 2 has a band, which is positive; sample 3 has a band, which is positive; and, sample 4 has a band, which is positive. It is indicated that the method provided by the present invention can accurately detect SARS-CoV-2 DNA and SARS-CoV-2 RNA with a detection accuracy of 100%.

### Embodiment 9 - detection of novel coronavirus (SARS-CoV-2) by clinical throat swab samples

9.1 - **T**hroat swab samples were collected from 5 suspected patients with novel coronavirus pneumonia, and the clinical nucleic acid diagnosis results were: sample 1, positive; sample 2, negative; sample 3, negative; sample 4, positive; and, sample 5, negative.

9.2 - Extract nucleic acids from throat swab samples.

9.3 - Water was used as a negative control group, 1000 copies of SARS-CoV-2 DNA fragments was used as a positive control group, and the nucleic acid of 5 patients obtained in step 9.2 was used as experimental groups, and the method of embodiment 8 was used for detection.

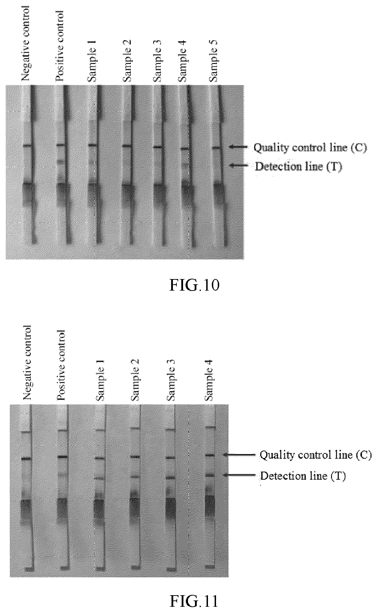

9.4 - Results: as shown in FIG. 10, all samples have bands at the position of the quality control line (C), indicating that the experimental results are valid. At the position of the detection line (T), negative control group has no band, which is negative; positive control group has a band, which is positive; sample 1 has a band, which is positive; sample 2 has no band, which is negative; sample 3 has no band, which is negative; sample 4 has a band, which is positive; and, sample 5 has no band, which is negative. The results of detecting suspected patients by the method provided by the present invention are completely consistent with the clinical detection results, indicating that the present invention has a potential of popularly application for detecting novel coronavirus.

### Embodiment 10 - detection of novel coronavirus (SARS-CoV-2) by clinical mouthwash samples

10.1 - Throat swab samples were collected from 4 suspected patients with novel coronavirus pneumonia, and the clinical nucleic acid diagnosis results were: sample 1, positive; sample 2, positive; sample 3, positive; and, sample 4, positive.

10.2 - Extract nucleic acids from throat swab samples.

10.3 - Water was used as a negative control group, 1000 copies of SARS-CoV-2 DNA fragments was used as a positive control group, and the nucleic acid of 4 patients obtained in step 10.2 was used as experimental groups, and the method of embodiment 8 was used for detection.

10.4 - Results: as shown in FIG. 11, all samples have bands at the position of the quality control line (C), indicating that the experimental results are valid. At the position of the detection line (T), negative control group has no band, which is negative; positive control group has a band, which is positive; sample 1 has a band, which is positive; sample 2 has a band, which is positive; sample 3 has a band, which is positive; and, sample 4 has a band, which is positive. 100% of confirmed patients can be detected by the method provided by the present invention. The detecting result is completely consistent with the clinical detection results, indicating that the present invention has the potential of popularly application for detecting novel coronavirus.

According to embodiments 1-10, the detection method for nucleic acid provided by the present invention is simple to operate, and does not need to rely on complex laboratory equipment, and can be detected in the wilderness field, at home, on-site, and so on. In addition, the method has accurate detection results for various nucleic acid detections such as pathogenic microorganism nucleic acid detection, viral nucleic acid detection, transgenic nucleic acid detection, genetic disease nucleic acid detection, tumor nucleic acid detection, DNA methylation nucleic acid detection, etc.

The above embodiments are merely illustrative of several implementation manners of the present disclosure, and the description thereof is more specific and detailed, but is not to be construed as a limitation to the patentable scope of the present disclosure. It should be pointed out that several variations and improvements can be made by those of ordinary skill in the art without departing from the conception of the present disclosure, but such variations and improvements should fall within the protection scope of the present disclosure. Therefore, the protection scope of the patent of the present disclosure should be subject to the appended claims.

## Claims

1. A method for detecting nucleic acid, **characterized in that** the method comprises the steps of:
S1, performing isothermal amplification: nucleic acid to be detected is subjected to an isothermal amplification;
S2, performing secondary amplification: the product of the isothermal amplification is subjected to a secondary amplification, and the product of the secondary amplification is a nucleic acid single strand;
S3, hybridizing with probe: the product of the secondary amplification is hybridized with a probe;
S4, degrading probe: a nuclease is added into the hybridized product to degrade redundant single-stranded nucleic acid; and,
S5, detecting product: the product obtained in step S4 is detected.

2. The method for detecting nucleic acid according to claim 1, wherein the method for the isothermal amplification in step S1 and the secondary amplification in step S2 comprises at least one of recombinase polymerase amplification, recombinase-aided amplification, loop-mediated isothermal amplification, strand displacement isothermal amplification, rolling circle amplification, nucleic acid sequence-based amplification, helicase-dependent isothermal DNA amplification and nicking endonuclease amplification reaction.

3. The method for detecting nucleic acid according to claim 2, wherein the method for the isothermal amplification in step S1 is recombinase polymerase amplification, and the method for the secondary amplification in step S2 is nicking endonuclease amplification reaction.

4. The method for detecting nucleic acid according to claim 3, wherein a nicking endonuclease used in the nicking endonuclease amplification reaction comprises at least one of Nb.BtsI,Nt.BspQI, Nt.CviPII, Nt.BstNBI, Nb.BsrDI, Nb.BtsI, Nt.AlwI, Nb.BbvCI, Nt.BbvCI, Nb.BsmI, Nb.BssSI and Nt.BsmAI; and, a strand displacement nucleic acid polymerase used in the nicking endonuclease amplification reaction comprises at least one of Bst3.0 DNA polymerase, Bst2.0 DNA polymerase, Klenow DNA polymerase and phi29 DNA polymerase.

5. The method for detecting nucleic acid according to claim 1, wherein the probe used in step S3 is a modified oligonucleotide probe.

6. The method for detecting nucleic acid according to claim 5, wherein the modification type of the probe comprises at least one of biotin modification, digoxigenin modification, fluorescein isothiocyanate modification, Texas red modification, phosphorylation modification, amino modification, sulfhydryl modification, thio modification, fluorescent group modification and quencher group modification.

7. The method for detecting nucleic acid according to claim 6, wherein the modification type of the probe comprises digoxigenin modification and biotin modification.

8. The method for detecting nucleic acid according to claim 1, wherein the nuclease used in step S4 comprises at least one of exonuclease VII, λ exonuclease, T5 exonuclease, T7 exonuclease, exonuclease V, exonuclease III, RecJ1 exonuclease and exonuclease I.

9. The method for detecting nucleic acid according to claim 1, wherein a product detecting method in step S5 comprises at least one of colloidal gold test strip method, colloidal carbon test strip method, fluorescent dye method, ultraviolet irradiation method, and real-time fluorescence quantitative PCR method.

10. The method for detecting nucleic acid according to claim 1, wherein the nucleic acid to be detected in the step S1 comprises at least one of DNA nucleic acid samples, RNA nucleic acid samples and cDNA nucleic acid samples reversely transcribed by RNA.

11. Use of the method according to any one of claims 1 to 10 in detecting pathogenic bacteria, viruses, transgenic nucleic acid, food components, genetic material variation and DNA methylation variation.

12. The use according to claim 11, wherein the pathogenic bacteria includes Salmonella, and the viruses includes African swine fever virus and novel coronavirus.

13. The use according to claim 11, wherein the detection of transgenic nucleic acid, food components, genetic material variation and DNA methylation variation includes detection of NOS gene, porcine mitochondrial CYTB gene, BRAF gene and Septin9 gene, respectively.

14. A kit for detecting nucleic acid, wherein comprising an isothermal amplification primer, a secondary amplification enzyme, a probe, and a nuclease.

15. The kit according to claim 14, wherein the probe is a modified oligonucleotide probe.

16. The kit according to claim 15, wherein the modification type of the probe comprises at least one of biotin modification, digoxigenin modification, fluorescein isothiocyanate modification, Texas red modification, phosphorylation modification, amino modification, sulfhydryl modification, thio modification, fluorescent group modification and quencher group modification.

17. The kit according to claim 16, wherein the nucleotide sequence of the probe comprises SEQ ID NO.3, SEQ ID NO.6, SEQ ID NO.9, SEQ ID NO. 12, SEQ ID NO. 15, SEQ ID NO. 18 and SEQ ID NO. 19.

18. The kit according to claim 14, wherein the nucleotide sequence of the isothermal amplification primer comprises SEQ ID NO.1, SEQ ID NO.2, SEQ ID NO.4, SEQ ID NO.5, SEQ ID NO.7, SEQ ID NO.8, SEQ ID NO. 10, SEQ ID NO.11, SEQ ID NO. 13, SEQ ID NO. 14, SEQ ID NO. 16, SEQ ID NO. 17, SEQ ID NO.20 and SEQ ID NO.21.

19. The kit according to claim 14, wherein the secondary amplification enzyme comprises at least one of a nicking endonuclease and a strand displacement nucleic acid polymerase, in which the nicking endonuclease comprises at least one of Nb.BtsI, Nt.BspQI, Nt.CviPII, Nt.BstNBI, Nb.BsrDI, Nb.BtsI, Nt.AlwI, Nb.BbvCI, Nt.BbvCI, Nb.BsmI, Nb.BssSI and Nt.BsmAI; and, the strand displacement nucleic acid polymerase comprises at least one of Bst3.0 DNA polymerase, Bst2.0 DNA polymerase, Klenow DNA polymerase and phi29 DNA polymerase.

20. The kit according to claim 14, wherein the nuclease comprises at least one of exonuclease VII, λ exonuclease, T5 exonuclease, T7 exonuclease, exonuclease V, exonuclease III, RecJ1 exonuclease and exonuclease I.

21. The kit according to claim 14, wherein further comprising a test strip which comprises a colloidal gold test strip or a colloidal carbon test strip.
